# EUROPEAN PATENT APPLICATION

(11) **EP 4 435 644 A1**
(43) Date of publication of application: **25.09.2024**
(21) Application number: 23305413.9
(22) Date of filing: 24.03.2023
(51) Int. Cl.: G06F 21/32, G02B 27/01

(54) **CALCULATION MODULE, SYSTEM WEARABLE DEVICE, EYEGLASSES AND COMPUTER-IMPLEMENTED METHOD FOR DETERMINING AN IDENTITY OF AN INDIVIDUAL**

(71) Applicant: Essilor International, 94220 Charenton-Le-Pont (FR)
(72) Inventor: AMIR, Bruno, 94100 SAINT MAUR DES FOSSES (FR); GIL, Paul, 80000 AMIENS (FR); SAHLER, Jean, 92160 ANTONY (FR)
(74) Representative: Korakis-Ménager, Sophie

(57) **Abstract**

Calculation module, system and computer-implemented method for determining an identity of an individual. The calculation module is configured to obtain a signal representing vibrations produced by heartbeats of a heart of the individual, to determine a representative segment of the signal and to compare the representative segment with a plurality of model segments to determine the identity of the individual, the model segments being associated respectively with model identities.

## Description

### Technical field

This disclosure relates to devices and methods for determining the identity of an individual.

### Background information and prior art

By identity of an individual one can understand the name of this individual or other elements about who this individual is.

One of the purposes of determining the identity of the individual may be to realize an authentication of the individual. The authentication of the individual may be used, for example, for entering a restricted area, paying using electronic payment or in computer science as a form of identification and access control, for example, for accessing protected data.

Another of the purposes of determining the identity of the individual may be to personalize the parameters of a device according to the preferences of the individual. The device which recognizes the individual may use the personal parameters of the recognized individual. For example, the device may personalize curves for tinting e-chromic lenses, for example, a speed of changing from one tint to another or a table associating a plurality of tints with a plurality of levels of ambient light. The device may also personalize parameters relating to sounds emitted by the device, such as the general level of the sounds or the level associated with different frequencies using an equalizer. It is also possible to personalize a welcome message, for example, displayed by a virtual reality or augmented reality headset.

To determine the identity of an individual, biometrics may be used. Biometrics are body measurements and calculations related to human characteristics. The biometrics are then measurable characteristics used to label and describe individuals.

Many different aspects of human physiology, chemistry or behavior can be used as biometrics. Among them some are related to the shape of a part of the body of the individual. Examples include, but are not limited to fingerprint, palm veins, face recognition, DNA, palm print, hand geometry, iris recognition, retina, odor/scent, voice, shape of ears and gait. However, these aspects of human physiology have drawbacks; DNA are too long to handle for real time application, fingerprint is sensible to alteration of the skin of the finger or the finger as such and voice recognition are sensible to diseases of the upper respiratory tract for example cold.

Thus, the goal of this disclosure is to provide methods and devices allowing the determination of the identity of an individual using biometrics that do not have the drawbacks of method of the state of the art.

### Summary

The following presents a simplified summary to provide a basic understanding of various aspects of this disclosure. This summary is not an extensive overview of all contemplated aspects and is intended to neither identify key or critical elements of all aspects nor delineate the scope of any or all aspects. The sole purpose is to present some concepts of one or more aspects in a simplified form as a prelude to the more detailed description that is presented later.

One aspect of this disclosure is a calculation module for determining an identity of an individual. The calculation module is configured to obtain a signal representing vibrations produced by heartbeats of a heart of the individual, to determine a representative segment of the signal and to compare the representative segment with a plurality of model segments to determine the identity of the individual, the model segments being associated respectively with model identities.

The signal representing vibrations produced by heartbeats is also called seismocardiogram (SCG). From the analysis of the signal representing vibrations produced by heartbeats, it is possible to obtain information on various mechanical events of the cardiac cycle including the opening and closing of the aortic and mitral valves, the atrial systole, isovolumic contraction and relaxation.

The period of the cardiac cycle during which the myocardium contracts is called systole and corresponds to first vibrations produced by the heart. The one during which it relaxes is the diastole and corresponds to second vibrations produced by the heart.

One knows, from the article of A.A. Bui, Z. Yu, and F. M. Bui, "A biometric modality based on the seismocardiogram (SCG)", published IEEE International Conference and Workshop on Computing and Communication (IEMCON), 2015, pp. 1-7, that the seismocardiogram presents characteristics unique to each individual.

Therefore, the calculation module of this disclosure allows a reliable determination of the identity of the individual. Furthermore, using heartbeats as biometrics allows a solution that is difficult to be stolen (compared to fingerprint and facial recognition), may be easily embedded in a frame of eyeglasses and allows the confirmation, at the same time as the identification, that the individual is alive.

Another aspect of this disclosure is a system comprising a wearable device and a calculation module for determining an identity of an individual wearing the wearable device, the wearable device comprising a sensor for determining a signal representing vibrations produced by cardiac cycles of a heart of the individual. The calculation module is configured to receive, from the sensor, a signal representing vibrations produced by heartbeats of a heart of the individual, to determine a representative segment of the signal and to compare the representative segment with a plurality of model segments to determine the identity of the individual, the model segments being respectively associated with model identities.

**Another** aspect of this disclosure is a wearable device comprising a calculation module for determining an identity of an individual wearing the wearable device. The wearable device comprises a sensor for determining a signal representing vibrations produced by cardiac cycles of a heart of the individual. The calculation module is configured to receive, from the sensor, a signal representing vibrations produced by heartbeats of a heart of the individual, to determine a representative segment of the signal and to compare the representative segment with a plurality of model segments to determine the identity of the individual, the model segments being respectively associated with model identities.

Another aspect of this disclosure is eyeglasses comprising a calculation module for determining an identity of an individual wearing the eyeglasses. The eyeglasses comprise a sensor for determining a signal representing vibrations produced by cardiac cycles of a heart of the individual. The calculation module is configured to receive, from the sensor, a signal representing vibrations produced by heartbeats of a heart of the individual, and to determine a representative segment of the signal and to compare the representative segment with a plurality of model segments to determine the identity of the individual, the model segments being respectively associated with model identities.

Another aspect of this disclosure is a computer implemented method for determining an identity of an individual. The method comprises obtaining a signal representing vibrations produced by heartbeats of a heart of the individual, determining a representative segment of the signal, and comparing the representative segment with a plurality of model segments to determine the identity of the individual, the model segments being respectively associated with model identities.

Another aspect of this disclosure is a computer program comprising instructions which, when the computer program is executed by a computer, cause the computer to carry out a method for determining an identity of an individual. The method comprises obtaining a signal representing vibrations produced by heartbeats of a heart of the individual, determining a representative segment of the signal, and comparing the representative segment with a plurality of model segments to determine the identity of the individual, the model segments being respectively associated with model identities.

**A** computer may include a memory and a processor. Examples of processors include microprocessors, microcontrollers, graphics processing units (GPUs), central processing units (CPUs), application processors, digital signal processors (DSPs), reduced instruction set computing (RISC) processors, systems on a chip (SoC), field-programmable gate arrays (FPGAs), programmable logic devices (PLDs), state machines, gated logic, discrete hardware circuits, and other suitable hardware configured to perform the various functionality described throughout this disclosure. The memory may be a computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor of the computer.

Another aspect of this disclosure is a non-transitory program storage device, readable by a computer, tangibly embodying a program of instructions executable by the computer to perform a method for determining an identity of an individual. The method comprises obtaining a signal representing vibrations produced by heartbeats of a heart of the individual, determining a representative segment of the signal, and comparing the representative segment with a plurality of model segments to determine the identity of the individual, the model segments being respectively associated with model identities.

### Description of the drawings

For a more complete understanding of the description provided herein and the advantages thereof, reference is now made to the brief descriptions below, taken in connection with the accompanying drawings and detailed description, wherein like reference numerals represent like parts.
Figure 1 represents the system for determining an identity of an individual.
Figure 2 represents eyeglasses, which are an example of the wearable device.
Figure 3 represents a removable clip attachable to the eyeglasses.
Figure 4 represents augmented reality eyeglasses, which are another example of the wearable device.
Figure 5 represents the method for determining an identity of the individual.
Figure 6-a to 6-d represent examples of seismocardiogram signal.
Figure 7 represents a first example of the step of determining a representative segment of the signal.
Figure 8 represents a second example of the step of determining a representative segment of the signal.

### Detailed description of embodiments

**The** detailed description set forth below in connection with the appended drawings is intended as a description of various possible embodiments and is not intended to represent the only embodiments in which the concepts described herein may be practiced. The detailed description includes specific details for the purpose of providing a thorough understanding of various concepts. However, it will be apparent to those skilled in the art that these concepts may be practiced without these specific details. In some instances, well-known structures and components are shown in block diagram form to avoid obscuring such concepts.

### Description of the system for determining an identity an individual

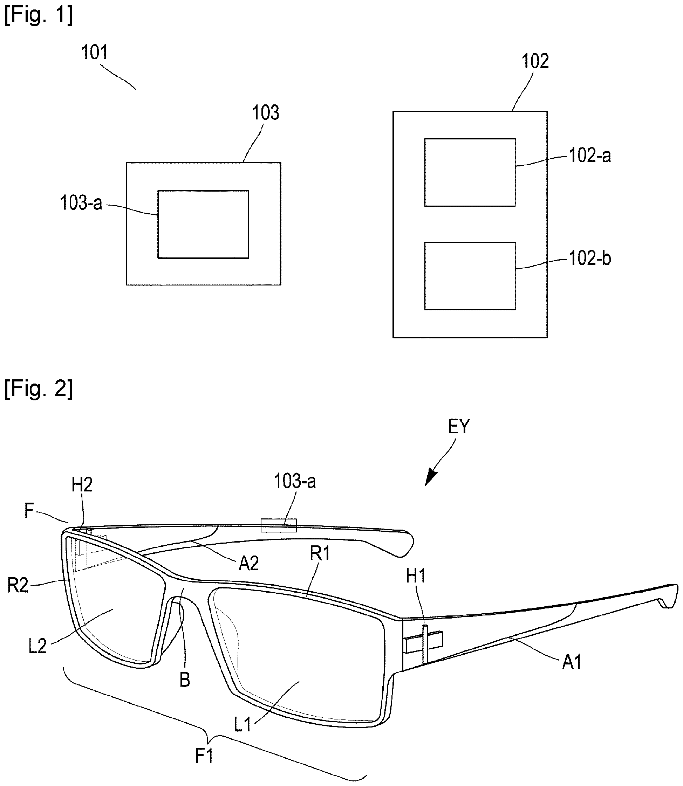

Figure 1 represents a system 101 for determining of the identity of the individual. The system 101 comprises a calculation module 102 and a wearable device 103. The calculation module 102 is configured to determine the identity of the individual wearing the wearable device 103. The wearable device comprises a sensor 103-a for determining a signal representing vibrations produced by heartbeats of a heart of the individual.

The calculation module 102 comprises a memory 102-a and a processor 102-b.

**The** calculation module may be a low resource calculation module.

Examples of processors 102-b include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionality described throughout this disclosure.

**The** memory 102-a is computer-readable media. By way of example, and not limitation, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by the processor 102-a of the calculation module 102.

**The** calculation module 102 may be included in independent module, for example, a smartphone or a computer, a system on chip (SoC) or a graphics processing unit (GPU). The calculation module 102 may also be a virtual machine located on a cloud network or a server not co-located with the patient or the system 101.

In other embodiments the calculation module 102 may be integrated to the wearable device 103 or may be attachable to the wearable device 103.

**The** system may also comprise a display unit. This display unit may be connected to the calculation module 102 uses to give instructions to the individual during the determination of its identity.

In embodiments the calculation module 102 may be part of the wearable device 103 for example headphones that can be circumaural headphones, supra-aural headphones, or in-ear headphones.

**The** figure 2 represents eyeglasses EY, which are an example of the wearable device 103. Eyeglasses are also known as eyewear. These eyeglasses EY comprise two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. The eyeglasses EY can be prescription eyeglasses for which the refraction as been adapted to the individual by an eye care professional and eyeglasses without refraction for example sunglasses.

**As** represented in the figure 2, the eyeglasses EY may integrate the sensor 103-a for determining a signal representing vibrations produced by cardiac cycles of a heart of the individual. The sensor 103-a may be located in one or both of the arms A1 and A2.

In embodiments the calculation module 102 may be integrated in the eyeglasses EY.

In other embodiments the calculation module 102 may be a module independent of the eyeglasses EY. In this case the calculation module 102 may be for example a smartphone.

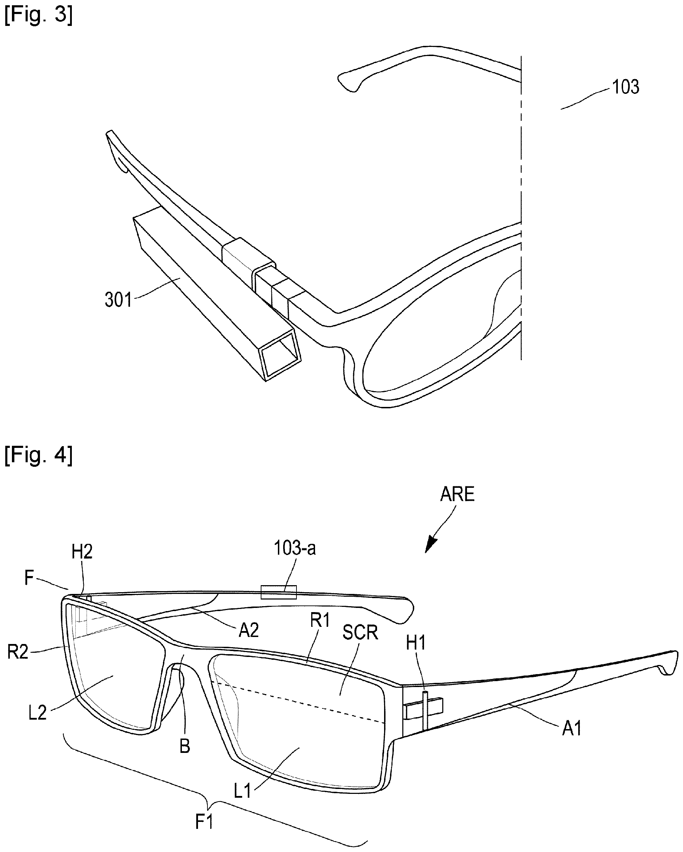

In other embodiments, represented figure 3, a removable clip 301 may be attached to the eyeglasses EY. This removable clip 301 may comprise the sensor 103-a and possibly the calculation module 102.

**The** figure 4 represents augmented reality eyeglasses ARE, which are another example of the wearable device 103. These augmented reality eyeglasses ARE comprise also two lenses L1 and L2 and a frame F. The frame F comprises two arms or temples A1 and A2 and a front part F1. The front part F1 comprises a right rim R1 and left rim R2 linked together by a bridge B. The front part F1 and the two arms A1 and A2 are linked using two hinges H1 and H2. The hinges H1 and H2 allow the individual to fold the arms A1 and A2 along the front part F1. The rims R1 and R2 of the frame F1 are configured to receive and to maintain the lenses L1 and L2. Furthermore, the augmented reality eyeglasses ARE comprise a screen SCR, generally a see-through display or transparent display.

**The** screen SCR may form the display unit usable to give instructions to the individual during the determination of its identity.

As represented in the figure 4, the augmented reality eyeglasses ARE may integrate the sensor 103-a for determining a signal representing vibrations produced by cardiac cycles of a heart of the individual. The sensor 103-a may be located in one or both of the arms A1 and A2.

As for the eyeglasses EY, in embodiments the calculation module 102 may be integrated in the augmented reality eyeglasses ARE.

As for the eyeglasses EY, in other embodiments, the calculation module 102 may be a module independent of the augmented reality eyeglasses ARE. In this case the calculation module 102 may be for example a smartphone.

As for the eyeglasses EY, in other embodiments, the removable clip 301 may be attached to the augmented reality eyeglasses ARE.

**The** wearable device may also be a watch, more precisely a wristwatch. Wristwatches are designed to be worn around the wrist, attached by a watch strap or other type of bracelet, including metal bands, leather straps, or any other kind of bracelet. In this case the watch may include the sensor 103-a and possibly the calculation module 102.

The watch may also be a smartwatch that is a wearable computer in the form of a watch. Modern smartwatches provide generally a local touchscreen interface for daily use, while an associated smartphone application and may comprise sensors used to determine physiological parameters of the individual. In this case the sensor 103-a may be one of these sensors. The calculation module 102 may be included in the smartwatch and may be share with other functions of the smartwatch.

The sensor 103-a of the wearable device 103 may be a MEMS sensor chosen among:
- a microphone
- an accelerometer
- a gyroscope
The sensor 103-a of the wearable device 103 may also be a capacitive sensor, a piezo sensor or an electrostatic sensor.

### Description of the method for determining the identity of the individual

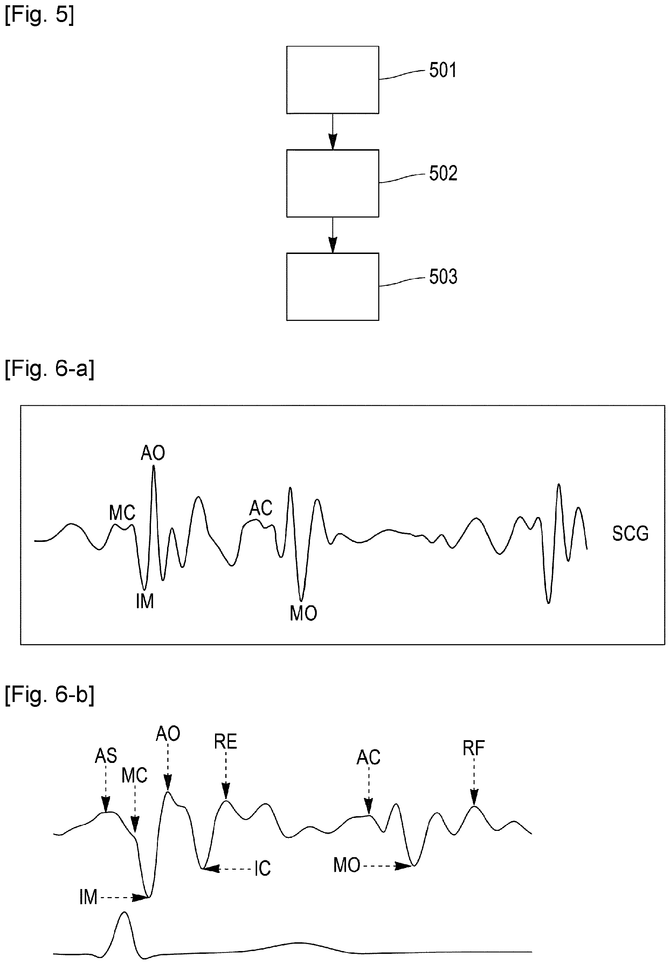

**To** realize this determination of an identity of an individual, the memory 102-a may store a computer program comprising instructions which, when the program is executed by the processor 102-b, cause the control module 102 to carry out a method, for example a computer implemented method, for determining an identity of the individual. The method, as presented in figure 5, comprises:
- a step 501 of obtaining a signal representing vibrations produced by heartbeats of a heart of the individual,
- a step 502 of determining a representative segment of the signal and
- a step 503 of comparing the representative segment with a plurality of model segments to determine the identity of the individual, the model segments being respectively associated with model identities.

During the step 501, the signal is obtained from the sensor 103-a of the wearable device 103.

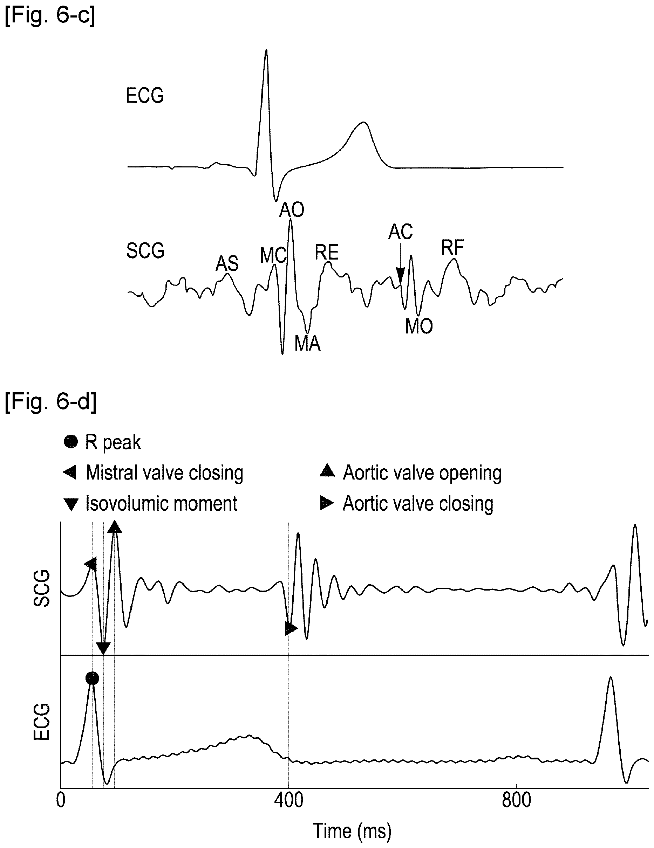

Examples of seismocardiogram are given in figures 6-a to 6-d. This signal is the measure of precordial vibrations produced by the beating heart, from which cardiac mechanics may be explored on a beat-to-beat basis. In this signal one may identify four fiducial points associated with the opening and closure of the aortic and mitral valves:
- AO (Aortic Opening),
- AC (Aortic Closure),
- MO (Mitral Opening) and
- MC (Mitral Closure).

Some other points of the signal are also of interest, among them one can cite:
- points of the signal corresponding to an isovolumic movement of a ventricle of the heart (IM),
- points of the signal corresponding to an isotonic contraction of a ventricle of the heart (IC),
- points of the signal corresponding to a rapid ventricular ejection (RE),
- points of maximum blood acceleration (MA),
- points of the signal corresponding to a rapid ventricular filing (RF).

In cardiac physiology, isometric contraction is an event occurring in early systole during which the ventricles contract with no corresponding volume change. This short-lasting portion of the cardiac cycle takes place while all heart valves are closed.

In an isotonic contraction, tension remains the same, whilst the muscle's length changes. Isotonic contractions differ from isokinetic contractions in that in isokinetic contractions the muscle speed remains constant.

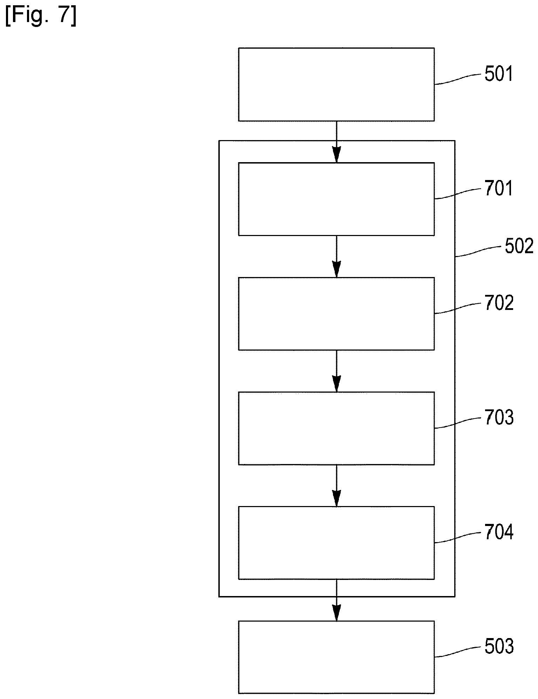

In a fist example represented figure 7 the step 502 of determining a representative segment of the signal may comprise:
- a step 701 of splitting the signal into a plurality of segments, each segment corresponding to one of the periods of a cardiac cycle,
- a step 702 of determining for each segment a characteristic point,
- a step 703 of synchronizing together the segments corresponding to a predetermined period of the cardiac cycle, the synchronization using the characteristic points, and
- a step 704 of averaging the synchronized segments to obtain the representative segment.

**The** cardiac cycle is the performance of the human heart from the beginning of one heartbeat to the beginning of the next. It consists of two periods: one during which the heart muscle relaxes and refills with blood, called diastole, following a period of robust contraction and pumping of blood, called systole. Pathologies such as heart failure can cause third periods to appear. In this case, one may delete these third periods from the signal used to realize the identification.

Using the first example one may split the signal in a plurality of segments. Each segment comprises one of the diastole parts of the cardiac cycle or one of the systole parts of the cardiac cycle. One may then select the segments of the systole parts or the segments of the diastole parts, synchronize these selected segments together and eventually average them.

**Generally,** one may use the following characteristic point to realize the synchronization:
- points of the segment corresponding to a closure of a mitral valve (MC),
- points of the segment corresponding to an isovolumic movement of a ventricle of the heart (IM),
- points of the segment corresponding to an opening of an aortic valve (AO),
- points of the segment corresponding to an isotonic contraction of a ventricle of the heart (IC),
- points of the segment corresponding to a rapid ventricular ejection (RE).

Remarkable points of systole are MC, IM, AO, IC, RE. It is advantageous to use point AO because using this point AO allows an easier and more robust way to do this identification.

Remarkable points of diastole are AC, MO, RF. It is advantageous to use point MO because this point is more characteristic of the diastole than other points.

Using the steps 701 to 704 one may determine a representative segment, that may be representative of the systolic parts or the diastolic parts of the cardiac cycle.

**After** this segmentation one may reject the too noisy segments. To realize this rejection one may:
- select the segments of the plurality of segments with a quality level above a quality threshold,
- determine a representative segment of the signal based only on the selected segments.

**The** thresholds may depend on the level of recognition requirement. If one needs a strong identification, then only the clearest segments may be selected. One may use one of the following metrics to determine the segments to select:
- SNR: Signal-To-Noise ratio of the segment.
- Relative band power of the estimated frequency in the cardiac frequency range.: this allows you to assess whether the frequency is alone in the heart frequency range. From 0.6 it's not bad, beyond 0.9 it's very good.
- PSNR: Peak Signal to noise ratio of the segment, generally if it is above 15 in linear one can select the segment.

In some embodiments and to improve the quality of the determination of the identity one may use the points AO, IC and/or RE to realize the synchronization of segments of the systolic periods. Advantageously the points AO can used if there is only one systole segment.

Regarding the diastolic periods one may use the part between points AC and MO to realize the synchronization. More precisely one can use the point MO to realize the synchronization.

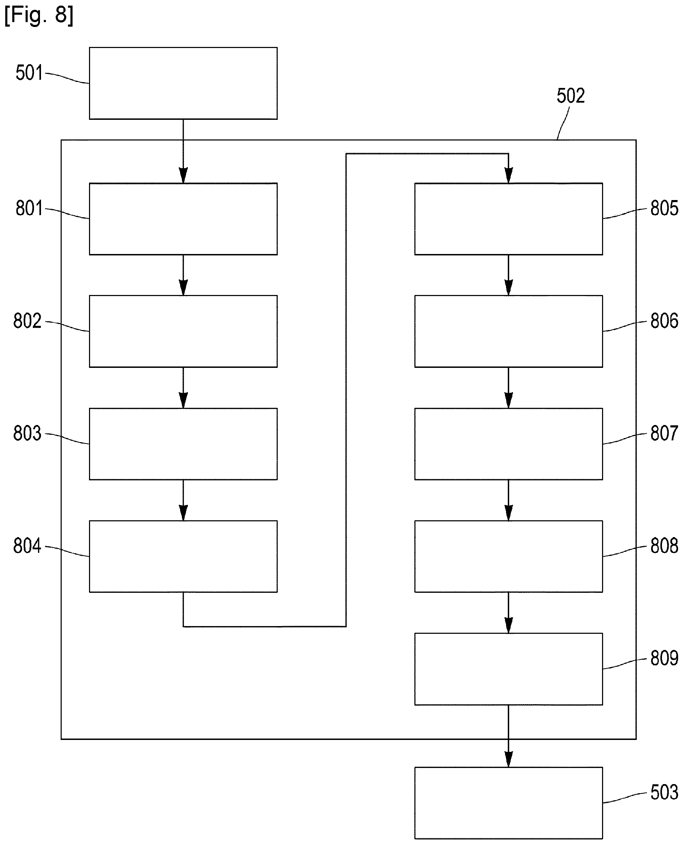

**The** figure 8 represents a second example of the step 502 of determining a representative segment. In this example the step 502 comprises:
- a step 801 of splitting the signal into a first plurality of first segments, each first segment corresponding to one of the periods of a cardiac cycle,
- a step 802 of determining for each first segment a first characteristic point,
- a step 803 of synchronizing together the first segments to obtain first synchronized segments, the synchronization using the first characteristic points,
- a step 804 of averaging the first synchronized segments to obtain a first averaged segment,
- a step 805 of splitting the signal into a second plurality of second segments, each second segment corresponding to another of the periods of a cardiac cycle,
- a step 806 of determining for each second segment a second characteristic point,
- a step 807 of synchronizing together the second segments to obtain second synchronized segments, the synchronization using the second characteristic points,
- a step 808 of averaging the second synchronized segments to obtain a second averaged segment and,
- a step 809 of concatenating the first averaged segment and the second averaged segment to obtain the representative segment.

In this second example, a first averaged segment corresponding for example to the systole parts and a second averaged segment corresponding for example to the diastole parts are concatenated together to obtain a more precise representative segment.

In other words, to improve the independent alignment of each part, the cross-correlation may be used to compute the lag between individual beats and the peak of the envelope. Aligning the beats, we may obtain "mean beats" or "median beats". Computing the new envelope of this median beats and the main peaks we may identify S1 peak and S2 peak and using one more time the cross-correlation, aligning the 2 mains complexes.

**To** realize the step 503 of comparing the representative segment with a plurality of model segments one may determine, for each of the model segments, a distance between the representative segment and the model segment. One may then determine the identity of the individual by selecting the identity associated with the model segment with minimal distance.

In embodiments the metric used to determine the distance may be chosen among:
- point-to-point distance between samples of the representative segment and one of the model segments,
- mutual information for example normalized mutual information between two segments,
- Hausdorff distance between two segments and,
- DTW (Dynamic time warping).

In other embodiments, to realize the step 503 of comparing the representative segment with a plurality of model segments one may use a convolutional neural network (CNN). This convolutional neural network may have been previously trained with a plurality of model identities associated respectively with a plurality of model representative segments.

In embodiments, one may provide an error message if the minimal distance is above a distance threshold. Generally the distance threshold depends on the metric used to compare the representative segment and one of the model segments. The values of the distances can depend on the amplitude, to avoid this one may normalize the curves to have thresholds that do not depend on the amplitudes of the signals (which can depend on the individual or the wearing conditions).

When the normalized mutual information (NMI) is used the value is close to 0 when two segments contain no information about one another, and NMI is close to 1 when two segments contain perfect information about one another. Depending on the "strength" of the identification, a threshold can be chosen between 0.2 and 0.8 or beyond. In this case the closer the NNI is from 1 the closer the two segments are and therefore the smaller the respective distance between the segments is.

When DTW is used, a value close to 0 indicates that the segments are very close. Therefore, depending the on the level of strength of the identification one can set a threshold close to 0.

When the Hausdorff distance is used a value close to 0 indicates that the two segments are very close. Therefore, depending the on the level of strength of the identification one can set a threshold close to 0.

### Description of use cases

In a first use case an individual would like to realize an action requiring the verification of its identity. This action may be for example entering a restricted area, paying using electronic payment or realizing an identification to access to protected data.

**The** individual may wear the wearable device 103, for example the eyeglasses EY presented figure 2 or 3 or the augmented reality eyeglasses ARE presented figure 4. The external system authorizing the action may communicate with the calculation module 102 to request the authentication of the individual. This communication may be realized for example using a wireless module using for example Wi-Fi wireless network protocol, cellular network protocol (for example 5G one) or the Bluetooth wireless technology standard.

Once the calculation module 102 received the request to authentic the individual, this calculation module 102 may start the method presented figure 5. Once the calculation module 102 determine the identity of the individual, the calculation module 102 may send the determined identity to the system authorizing the action.

**The** external system may authorize or not the action according to the received determined identity and values of other parameters internal to this system.

As in the first use case, in a second use case an individual would also like to realize an action requiring the verification of its identity. The type of the action may be similar to the ones of the first use case.

As in the first use case, the individual may wear the wearable device 103, for example the eyeglasses EY presented figure 2 or 3 or the augmented reality eyeglasses ARE presented figure 4. The external system authorizing the action may communicate with the calculation module 102 to request the authentication of the individual. This communication may be realized for example using a wireless module using for example Wi-Fi wireless network protocol, cellular network protocol (for example 5G one) or the Bluetooth wireless technology standard.

Differently form the first use case, once the calculation module 102 received the request to authentic the individual, the calculation module 102 may determine a time of a last determination of the identity of the individual. When a duration between the time of the last determination and the actual time is below a duration threshold and the individual wore continuously the wearable device, the calculation module may transmit the identity determined during the last determination. When the duration is above the duration threshold, or the individual did not wear continuously the wearable device, the calculation module may determine the identity of the individual on the spot and send the newly determined identity to the system authorizing the action.

In other words, to realize this second use case, the method of the figure 5 may also comprise:
- a step of receiving a request for the identification of the individual,
- a step of determining a time of a last determination of the identity of the individual,
when a duration between the time of the last determination and the actual time is below a duration threshold, the calculation module is configured to transmit the identity determined during the last determination.

**The** external system may authorize or not the action according to the received newly determined identity and values of other parameters internal to this system.

In an embodiment the system 101 and more precisely the calculation module 102 may be configured to determine a confidence level of the last determined identity. If the confidence level if above a predetermined (and the other previously conditions are respected) the calculation module is configured to transmit the identity determined during the last determination.

If at least one of the previously presented conditions is not respected, the calculation module is configured to determine the identity of the individual.

In an embodiment, the device requesting the identity verification may require a minimum level of identification, for example using a strong identification.

Independently from the first use case or the second case or in addition to these use cases, in a third use case the individual would like to have an automatic configuration of the external system or the wearable device 103 according to the determined identity.

**The** individual may wear the wearable device 103, for example the eyeglasses EY presented figure 2 or 3 or the augmented reality eyeglasses ARE presented figure 4. The external system authorizing the action may communicate with the calculation module 102 to request the authentication of the individual. This communication may be realized for example using a wireless module using for example Wi-Fi wireless network protocol, cellular network protocol (for example 5G one) or the Bluetooth wireless technology standard.

Once the calculation module 102 received the request to authentic the individual, this calculation module 102 may start the method presented figure 5. Once the calculation module 102 determine the identity of the individual, the calculation module 102 may send the determined identity to the system authorizing the action.

**The** external system or the wearable device 103 may then be automatically configured to fulfill the requirements of the individual. For example, the wearable device 103 may personalize curves for tinting e-chromic lenses. The device may also personalize parameters relating to sounds emitted by the device, such as the general level of the sounds or the level associated with different frequencies using an equalizer. It is also possible to personalize a welcome message, for example, displayed by a virtual reality or augmented reality headset.

## Claims

1. Calculation module (102) for determining an identity of an individual, the calculation module (102) being configured:
• to obtain a signal representing vibrations produced by heartbeats of a heart of the individual,
• to determine a representative segment of the signal and
• to compare the representative segment with a plurality of model segments to determine the identity of the individual, the model segments being associated respectively with model identities.

2. Calculation module (102) according to the claim 1, the calculation module (102) being configured to determine the representative segment by:
• splitting the signal into a plurality of segments, each segment corresponding to one of the periods of a cardiac cycle,
• determining for each segment a characteristic point,
• synchronizing together the segments corresponding to a predetermined period of the cardiac cycle, the synchronization using the characteristic points, and
• averaging the synchronized segments to obtain the representative segment.

3. Calculation module (102) according to the claim 1, the calculation module (102) being configured to determine the representative segment by:
• splitting the signal into a first plurality of first segments, each first segment corresponding to one of the periods of a cardiac cycle,
• determining for each first segment a first characteristic point,
• synchronizing together the first segments to obtain first synchronized segments, the synchronization using the first characteristic points,
• averaging the first synchronized segments to obtain a first averaged segment,
• splitting the signal into a second plurality of second segments, each second segment corresponding to another of the periods of a cardiac cycle,
• determining for each second segment a second characteristic point,
• synchronizing together the second segments to obtain second synchronized segments, the synchronization using the second characteristic points,
• averaging the second synchronized segments to obtain a second averaged segment, and
• concatenating the first averaged segment and the second averaged segment to obtain the representative segment.

4. Calculation module (102) according to the claim 2 or 3, the calculation module (102) being configured:
• to select the segments of the plurality of segments with a quality level above a quality threshold,
• to determine a representative segment of the signal based only on the selected segments.

5. Calculation module (102) according to any one of the claims 1 to 4, the calculation module (102) being also configured to determine, for each of the model segments, a distance between the representative segment and the model segment, the identity of the individual being the identity associated with the model segment with minimal distance.

6. Calculation module (102) according to any one of the claims 2 to 5,
the characteristic point being chosen among:
• a point of the segment corresponding to a closure of a mitral valve (MC),
• a point of the segment corresponding to an isovolumic movement of a ventricle of the heart (IM),
• a point of the segment corresponding to an opening of an aortic valve (AO),
• a point of the segment corresponding to an isotonic contraction of a ventricle of the heart (IC) and
• a point of the segment corresponding to a rapid ventricular ejection (RE).

7. Calculation module (102) according to any one of the claims 1 to 6, the calculation module (102) being configured:
• to receive a request for the identification of the individual,
• to determine a time of a last determination of the identity of the individual,
when a duration between the time of the last determination and the actual time is below a duration threshold, the calculation module is configured to transmit the identity determined during the last determination.

8. System (101) comprising a wearable device (103) and a calculation module (102) for determining an identity of an individual wearing the wearable device (103), the wearable device (103) comprising a sensor (103-a) for determining a signal representing vibrations produced by cardiac cycles of a heart of the individual,
the calculation module (102) being configured:
• to receive, from the sensor, a signal representing vibrations produced by heartbeats of a heart of the individual,
• to determine a representative segment of the signal and
• to compare the representative segment with a plurality of model segments to determine the identity of the individual, the model segments being respectively associated with model identities.

9. System (101) according to the claim 8,
the sensor (103-a) of the wearable device (103) being a MEMS sensor chosen among:
• a microphone
• an accelerometer
• a gyroscope
or the sensor (103-a) of the wearable device (103) being a capacitive sensor, a piezo sensor or an electrostatic sensor.

10. System (101) according to the claim 8 or 9, the calculation module (102) being configured:
• to receive a request for the identification of the individual,
• to determine a time of a last determination of the identity of the individual,
when a duration between the time of the last determination and the actual time is below a duration threshold and the individual wore continuously the wearable device, the calculation module is configured to transmit the identity determined during the last determination.

11. Wearable device (103) comprising a calculation module (102) for determining an identity of an individual wearing the wearable device (103), the wearable device (103) comprising a sensor (103-a) for determining a signal representing vibrations produced by cardiac cycles of a heart of the individual,
the calculation module (102) being configured:
• to receive, from the sensor, a signal representing vibrations produced by heartbeats of a heart of the individual,
• to determine a representative segment of the signal and
• to compare the representative segment with a plurality of model segments to determine the identity of the individual, the model segments being respectively associated with model identities.

12. Eyeglasses (EY) comprising a calculation module (102) for determining an identity of an individual wearing the eyeglasses (EY), the eyeglasses (EY) comprising a sensor (103-a) for determining a signal representing vibrations produced by cardiac cycles of a heart of the individual,
the calculation module (102) being configured:
• to receive, from the sensor, a signal representing vibrations produced by heartbeats of a heart of the individual,
• to determine a representative segment of the signal and
• to compare the representative segment with a plurality of model segments to determine the identity of the individual, the model segments being respectively associated with model identities.

13. A computer-implemented method for determining an identity of an individual
the method comprising:
• obtaining (501) a signal representing vibrations produced by heartbeats of a heart of the individual,
• determining (502) a representative segment of the signal and,
• comparing (503) the representative segment with a plurality of model segments to determine the identity of the individual, the model segments being respectively associated with model identities.

14. The computer-implemented method according to the claim 13, the representative segment being determined by:
• splitting (701) the signal into a plurality of segments, each segment corresponding to one of the periods of a cardiac cycle,
• determining (702) for each segment a characteristic point,
• synchronizing (703) together the segments corresponding to a predetermined period of the cardiac cycle, the synchronization using the characteristic points, and
• averaging (704) the synchronized segments to obtain the representative segment.

15. The computer implemented method according to the claim 13, the representative segment being determined by:
• splitting (801) the signal into a first plurality of first segments, each first segment corresponding to one of the periods of a cardiac cycle,
• determining (802) for each first segment a first characteristic point,
• synchronizing (803) together the first segments to obtain first synchronized segments, the synchronization using the first characteristic points, and
• averaging (804) the first synchronized segments to obtain a first averaged segment,
• splitting (805) the signal into a second plurality of second segments, each second segment corresponding to another of the periods of a cardiac cycle,
• determining (806) for each second segment a second characteristic point,
• synchronizing (807) together the second segments to obtain second synchronized segments, the synchronization using the second characteristic points, and
• averaging (808) the second synchronized segments to obtain a second averaged segment,
• concatenating (809) the first averaged segment and the second averaged segment to obtain the representative segment.
